# EUROPEAN PATENT SPECIFICATION

(11) **EP 3 203 227 B1**
(45) Date of publication and mention of the grant of the patent: **13.07.2022**
(21) Application number: 14903169.2
(22) Date of filing: 30.09.2014
(51) Int. Cl.: G01N 30/02, G01N 30/62, G01N 33/48

(54) **CHRONIC HEART DISEASE PATIENT SPECIFIC BIOMARKER COMPOSITION AND USE THEREOF**
FÜR PATIENTEN MIT CHRONISCHER HERZERKRANKUNG SPEZIFISCHE BIOMARKERZUSAMMENSETZUNG UND VERWENDUNG DAVON
COMPOSITION DE BIOMARQUEUR SPÉCIFIQUE D'UN PATIENT ATTEINT D'UNE MALADIE CARDIAQUE CHRONIQUE ET SON UTILISATION

(43) Date of publication of application: 09.08.2017
(73) Proprietor: BGI Shenzhen Co., Limited, Shenzhen, Guangdong 518083 (CN); BGI Shenzhen, Shenzhen, Guangdong 518083 (CN)
(72) Inventor: FENG, Qiang, Shenzhen, Guangdong 518083 (CN); LIU, Zhipeng, Shenzhen, Guangdong 518083 (CN); MENG, Nan, Shenzhen, Guangdong 518083 (CN); WANG, Jun, Shenzhen, Guangdong 518083 (CN)
(74) Representative: Inspicos P/S
(86) International application number: PCT/CN2014/087853
(87) International publication number: WO 2016/049830

(56) References cited:
- WO-A2-2013/171586
- CN-A- 101 769 910
- CN-A- 102 323 351
- HALEEM JI ET AL.: 'Detection of Bladder Cancer in Human Urine by Metabolomic Profiling Using High Performance Liquid Chromatography/Mass Spectrometry' THE JOURNA OF UROLOGY vol. 179, no. 6, 01 June 2008, pages 2422 - 2426, XP022650280 DOI: 10.1016/J.JURO.2008.01.084

## Description

### Technical Field

The present invention relates to a disease-specific metabolite profile, and particularly to a biomarker composition obtained by screening from blood plasma-specific metabolite profiles of coronary heart disease subjects. The present invention also relates to a use of the biomarker compositions in risk assessment, diagnosis, early diagnosis, or pathological staging of coronary heart disease, and to a method for risk assessment, diagnosis, early diagnosis, or pathological staging of coronary heart disease.

### Background Art

Coronary artery heart disease (CAHD), also known as ischemic heart disease, or coronary heart disease for short, is one of the most common heart diseases, referring to dysfunctions and/or organic pathologic changes of cardiac muscles caused by coronary artery stenosis or insufficient blood supply, thus it is also called as ischemic heart disease (IHD). In 2012, it is the first cause of death in the world^{[1]}, and one of the major reasons for hospitalization^{[2]}. Coronary heart disease may occur at any age, even in children, but the major age of onset is middle age, and its incidence increases with age. Nearly 17 million people die from atherosclerotic heart diseases every year in the world, and it is estimated that there is an increase of 50% in deaths by 2020, reaching 25 million per year, accounting for 1/3 of deaths in the world. In China, there are 2.5 million people die from cardiovascular diseases per year; the new myocardial infarctions occur in 500,000 people per year; the occurrence of coronary heart disease has significant regional differences, that is, it is generally higher in the northern cities than the southern cities; there are also significant gender differences, that is, the ratio of men to women is 2-5: 1. The data show that there are also similar differences in distribution of coronary heart disease in patients in the world^{[3]}. At present, the diagnosis of coronary heart disease still lacks a uniform standard, and the existing diagnostic methods such as electrocardiogram, electrocardiogram stress test, dynamic electrocardiogram, radionuclide myocardial imaging, echocardiography, hematological examination, coronary CT, coronary angiography and intravascular imaging techniques all have some shortcomings. For example, the observation of symptoms, echocardiography and so on have strong subjectivity, the coronary CT, coronary angiography and intravascular imaging techniques are invasive diagnosis which cause additional pains in patients. The diagnosis using the single markers that have been found in blood has disadvantages such as poor sensitivity and specificity, and high false positive rate. It is of great significance to develop a noninvasive, specific and accurate method for the diagnosis of coronary heart disease^{[4,5]}.

Metabolomics is a systematic biology discipline developed after genomics and proteomics to study the species, quantities and variations of endogenous metabolites in a subject after affections of internal or external factors. Metabolomics is to analyze the whole metabolic profile of an organism, and to explore the corresponding relationships between metabolites and physiological and pathological changes, so as to provide a basis for the diagnosis of diseases. Therefore, it is of great significance to screen metabolic markers associated with coronary heart disease, in particular to use a combination of multiple metabolic markers, for the metabolomics research, clinical diagnosis and treatment of coronary heart disease.

### Contents of the Invention

Aiming at the shortcomings such as trauma and invasion of the existing diagnostic methods for coronary artery diseases, the problem to be solved by the present invention is to provide a biomarker combination (i.e., a biomarker composition) that can be used for the diagnosis and risk assessment of coronary heart disease, and a method for diagnosis and risk assessment of coronary heart disease.

In the present invention, liquid chromatography-mass spectrometry is used for analyzing the metabolite profiles of blood plasma samples of the coronary heart disease group and the control group, and pattern recognition is used for analyzing and comparing the metabolite profiles of the coronary heart disease group and the control group, so as to determine specific liquid chromatography-mass spectrometry data and corresponding specific biomarkers, which provide a basis for the subsequent theoretical research and clinical diagnosis.

Disclosed is a set of biomarkers including the following Biomarkers 1 to 6:
Biomarker 1, which has a mass-to-charge ratio of 310.04 ± 0.4amu, and a retention time of 611.25 ± 60s;
Biomarker 2, which has a mass-to-charge ratio of 311.05 ± 0.4amu, and a retention time of 611.26 ± 60s;
Biomarker 3, which has a mass-to-charge ratio of 220.00 ± 0.4amu, and a retention time of 122.77 ± 60s;
Biomarker 4, which has a mass-to-charge ratio of 247.09 ± 0.4amu, and a retention time of 146.37 ± 60s;
Biomarker 5, which has a mass-to-charge ratio of 255.03 ± 0.4amu, and a retention time of 117.92 ± 60s; and
Biomarker 6, which has a mass-to-charge ratio of 170.03 ± 0.4amu, and a retention time of 202.18 ± 60s;
for example, comprising 1, 2, 3, 4, 5 or 6 of these biomarkers.

The characteristics of the above six biomarkers are shown in Table 1.

Also disclosed are training set data for the contents of the biomarker composition in a coronary heart disease subject and a normal subject.

In one embodiment of the present invention, the training set data are shown in Table 2.

The present invention relates to a method for risk assessment, diagnosis, early diagnosis or pathological staging of coronary heart disease, comprising a step of determining content of each biomarker of the biomarker composition in a sample (e.g., blood plasma, whole blood) of a subject.

The method of the present invention is disclosed in claim 1.

In the method of the present invention, a liquid chromatography-mass spectrometry method is used for determining the content of each biomarker of the biomarker composition in the sample (blood plasma, whole blood) of the subject.

According to the present invention, the method further comprises a step of establishing a training set for contents of the biomarker composition in samples (blood plasma, whole blood) of a coronary heart disease subject and a normal subject (control group).

Typically the training set is established by using a multivariate statistical classification model (e.g., a random forest model).

The training set may comprise data as shown in Table 2.

According to the present invention, the method further comprises a step of comparing the content of each biomarker of the biomarker composition in the sample (blood plasma, whole blood) of the subject to the data of training set of the biomarker compositions of the coronary heart disease subject and the normal subject.

Typically the training set is established by using a multivariate statistical classification model (e.g., a random forest model).

The step of comparing the content of each biomarker is carried out by using a receiver operating characteristic curve (ROC).

Finally, the results obtained in the ROC curve are interpreted. The step of interpreting the ROC curve may be carried out by a method comprising:
if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patent with coronary heart disease.

In a particular embodiment of the present invention, the method comprises the steps of:
1) determining the content of each biomarker of the biomarker composition in blood plasma of a subject by means of liquid chromatography-mass spectrometry;
2) determining the content of the biomarker composition in blood plasma of a coronary heart disease subject and a normal subject by means of liquid chromatography-mass spectrometry, and establishing a training set (for example, as shown in Table 2) for the content of the biomarker composition by using a random forest model;
3) comparing the content of each biomarker of the biomarker composition in blood plasma of the subject to the data of the training set of the biomarker composition of the coronary heart disease subject and the normal subject by using ROC curves;
4) if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patent with coronary heart disease.

The biomarker composition , is used in risk assessment, diagnosis, early diagnosis or pathological staging of coronary heart disease.

A liquid chromatography-mass spectrometry method is used for determining the content of each biomarker of the biomarker composition in the sample (blood plasma, whole blood) of the subject.

The present invention further comprises a step of establishing a training set for content of each biomarker of the biomarker composition of a coronary heart disease subject and a normal subject.

In one embodiment of the present invention, the training set is established by using a multivariate statistical classification model (e.g., a random forest model).

In one embodiment of the present invention, the training set comprises data as shown in Table 2.

The method of the present invention further comprises a step of comparing the content of each biomarker of the biomarker composition in the sample (blood plasma, whole blood) of the subject to the data of training set for the biomarker composition of the coronary heart disease subject and the normal subject.

In one embodiment of the present invention, the training set is established by using a multivariate statistical classification model (e.g., a random forest model).

In one embodiment of the present invention, the training set comprises data as shown in Table 2.

In the present invention, the comparing is a method using a receiver operating characteristic curve for comparison.

In one embodiment of the present invention, the result is interpreted by a method comprising: if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patent with coronary heart disease.

In an embodiment of the invention, the content of each biomarker in the biomarker composition and the data of content of each biomarker in the training set are obtained by the following steps:
(1) collection and treatment of samples: a blood plasma sample is collected from a clinical patient or a model animal;
   the sample is subjected to process, such as liquid-liquid extraction using an organic solvent, wherein the organic solvent includes, but is not limited to, ethyl acetate, chloroform, diethyl ether, n-butanol, petroleum ether, dichloromethane, acetonitrile, etc.; or protein precipitation, wherein the protein precipitation comprising precipitation of adding an organic solvent (such as methanol, ethanol, acetone, acetonitrile, isopropyl alcohol), various acid, alkali or salt precipitation, heating precipitation, filtration/ultrafiltration, solid-phase extraction, centrifugation, in single or comprehensive manner;
   the sample is dried or not dried, and then dissolved in an organic solvent (e.g., methanol, acetonitrile, isopropanol, chloroform, etc., preferably methanol, acetonitrile) or water (in single or combination, with or without salt);
   and then the sample is not derivatized or derivatized with a reagent (e.g., trimethylsilane, ethyl chloroformate, N-methyltrimethylsilyl trifluoroacetamide, etc.).
(2) liquid chromatography-mass spectrometry (HPLC-MS): a metabolite profile of blood plasma is obtained by liquid chromatography and mass spectrometry, the metabolite profile is processed to obtain data of each peak such as peak height or peak area (peak intensity), mass-to-charge ratio and retention time, in which the peak area represents biomarker content.

In a particular embodiment of the present invention, the treatment in step (1) comprises the following step: the sample is subjected to liquid-liquid extraction with an organic solvent; or to protein precipitation; the sample is dried or not dried, and then dissolved in single or combination of organic solvents or water, the water is free of salt or contains a salt, and the salt comprises sodium chloride, phosphate, carbonate and the like; the sample is not derivatized or derivatized with a reagent.

In a specific embodiment of the present invention, in the liquid-liquid extraction with organic solvent in step (1), the organic solvent includes, but is not limited to, ethyl acetate, chloroform, diethyl ether, n-butanol, petroleum ether, dichloromethane, acetonitrile.

In a particular embodiment of the invention, the protein precipitation in step (1) comprises, but is not limited to, precipitation of adding an organic solvent, or various acid, alkali or salt precipitation, heating precipitation, filtration/ultrafiltration, solid phase extraction, centrifugation in single or combination manner, in which the organic solvent comprises methanol, ethanol, acetone, acetonitrile, isopropanol.

In a specific embodiment of the present invention, step (1) preferably comprises performing the treatment by using a protein precipitation method, preferably a protein precipitation using ethanol.

In a specific embodiment of the present invention, in step (1), the sample is dried or not dried, and then dissolved in an organic solvent or water; the organic solvent includes methanol, acetonitrile, isopropanol, chloroform, preferably methanol, or acetonitrile.

In a specific embodiment of the present invention, in step (1), the sample is derivatized with a reagent, the reagent comprises trimethylsilane, ethyl chloroformate, N-methyltrimethylsilyl trifluoroacetamide.

In a specific embodiment of the present invention, in step (2), the metabolite profile is processed to obtain raw data, the raw data are preferably data of peak height or peak area, as well as mass number and retention time of each peak.

In a specific embodiment of the present invention, in step (2), the raw data are subjected to peak detection and peak matching, the peak detection and the peak matching are preferably performed by using XCMS software.

The mass spectrometry types are roughly divided into four types including ion trap, quadrupole, electrostatic field orbital ion trap, and time-of-flight mass spectrometries, and the mass deviations of these four types are 0.2 amu, 0.4 amu, 3 ppm and 5 ppm, respectively. The experimental results in the present invention are obtained by ion trap analysis, and therefore suitable for all mass spectrometric instruments using ion trap and quadrupole as mass analyzers, including Thermo Fisher's LTQ Orbitrap Velos, Fusion, Elite et al., Waters' TQS, TQD, etc. , AB Sciex 5500, 4500, 6500, etc., Agilent's 6100, 6490, Bruker's amaZon speed ETD and so on.

In an embodiment of the present invention, the content of biomarker is expressed by peak area (peak intensity) of mass spectrum.

In the present invention, the mass-to-charge ratio and the retention time have the meanings in the art.

It is well known to those skilled in the art that the atomic mass unit and retention time of each biomarker of the biomarker composition of the present invention will fluctuate within certain ranges when different liquid chromatography-mass spectrometry devices and different detection methods are employed; wherein the atomic mass unit may fluctuate within a range of ± 0.4 amu, for example ± 0.2 amu, for example ± 0.1 amu, and the retention time may flucturate within a range of ± 60 s, for example ± 45 s, for example ± 30 s, for example ± 15 s.

In the present invention, the methods of using the random forest model and the ROC curves are well known in the art (see the references [7] and [8]), and those skilled in the art can set and adjust parameters according to specific situations.

In the present invention, the training set and test set have the meanings well known in the art. In an embodiment of the invention, the training set refers to a data set of contents for biomarkers in samples of coronary heart disease subjects and normal subjects having given numbers. The test set is a set of data used to test the performance of the training set.

In the present invention, a training set of biomarkers of coronary heart disease subjects and normal subjects is constructed, and the content values of biomarkers of test samples are evaluated using the training set as basis.

In the present invention, the unit of mass-to-charge ratio is amu, amu refers to atomic mass unit, also known as Dalton (Da, D), which is a unit used to measure atomic or molecular mass, and is defined as 1/12 of atomic mass of C-12.

In the present invention, one or more of the biomarkers may be used for risk assessment, diagnosis or pathological staging, etc., of coronary heart disease, preferably at least four of them, i.e., Biomarkers 1 to 3 and Biomarker 6, are used for evaluation, or all of the six biomarkers (i.e., Biomarkers 1 to 6) are used for evaluation, so as to obtain desired sensitivity and specificity.

Those skilled in the art would understand that when sample size is further expanded, the normal content value interval (absolute value) of each biomarker in the sample can be obtained using sample detection and calculation methods known in the art. In this way, when the content of the biomarker is detected by methods other than mass spectrometry (for example, by using an antibody and an ELISA method), the absolute value of the detected biomarker content can be compared with the normal content value, optionally, risk assessment, diagnosis or pathological staging, etc., of coronary heart disease can also be achieved in combintion with statistical methods.

Without being bound by any theory, the inventors have pointed out that these biomarkers are endogenous compounds present in human body. The metabolite profile of blood plasma of a subject is analyzed by the method of the present invention, and the mass value and the retention time in the metabolite profile indicate the presence and the corresponding position of the corresponding biomarker in the metabolite profile. At the same time, the biomarkers of coronary heart disease population exhibit certain content ranges in their metabolite profiles.

Endogenous small molecules in body are the basis of life activities, and changes of disease states and body functions will inevitably lead to changes of metabolism of the endogenous small molecules in the body. The present invention shows that there are significant differences in blood plasma metabolite profiles between the coronary heart disease group and the control group. In the present invention, a plurality of relevant biomarkers are obtained through comparison and analysis of metabolite profiles of the coronary heart disease group and the control group, which can be used in combintion with high quality data of metabolite profiles of biomarkers of coronary heart disease population and normal population as the training set to accurately perform risk assessment, early diagnosis and pathological staging of coronary heart disease. Compared with the commonly used diagnostic methods, this method has advantages of noninvasion, convenience and rapid, and has high sensitivity and good specificity.

### Brief Description of the Drawings

Fig.1 shows total ion chromatograms of mass spectrometry for coronary heart disease group (a) and normal group (b).
Fig.2 shows PLS-DA score plots, in which prisms (white) represent normal group, triangles (black) represent coronary heart disease group.
Fig.3 shows a loading-plot of principal components, in which triangles (black) represent variables with VIP value greater than 1.
Fig.4 shows a Volcano-plot, in which differential metabolites are located above the horizontal dotted line, wherein the materials (black triangles) on the ambilateral sides of the two vertical dashed lines are metabolites with fold-change greater than 1.2 and Q-value less than 0.05, and the materials (gray spheres) between the two vertical dashed lines are metabolites with fold-change less than 0.8 and Q-value less than 0.05.
Fig.5 shows S-plot, in which prisms (black) represent variables with VIP greater than 1.
Fig.6 shows score graphs for analysis of principal components, in which prisms (white) represent the normal group, triangulars (black) represent the coronary heart disease group, and the analysis of principal components is performed by analyzing 83 test set data using the disclosed markers.
Fig.7 shows ROC diagram of random forest model (Random forest model), in which Training ROC (Fig.7a) is based on the training set, AUC = 1; and Test ROC (Fig.7b) is based on the test set, AUC = 1.
Fig.8 shows ROC test set diagram, in which mass-to-charge ratios 310.04 and 311.05 are randomly removed from the training set, AUC = 0.8851.
Fig.9 shows diagram for random combinations of 6 potential markers, in which the left side of the vertical line mark gives 4 markers that need to be tested at least.

### Specific Models for Carrying Out the Invention

While the embodiments of the present invention will be described in detail with reference to the following examples, it will be understood by those skilled in the art that the following examples are intended to be illustrative of the invention and are not to be taken as limiting the scope of the invention. In the examples, when specific conditions are not given, conventional conditions or conditions recommended by the manufacturer are employed. The used reagents or instruments which manufacturers are not given are all conventional products commercially available in the markets.

The blood plasma samples of coronary heart disease and normal subjects in the present invention are from the Guangdong General Hospital.

### Example 1

1.1 Collection of samples: morning blood samples of volunteers were collected, immediately placed and stored in -80°C low temperature refrigerator. A total of 52 blood samples were collected from the normal group and 40 blood samples were collected from the coronary heart disease group.

1.2 Treatment of samples: frozen samples were thawed at room temperature, 500 µL of each blood plasma sample was taken and placed in 2.0 mL centrifuge tube, added with 1000 µL of methanol for dilution, centrifuged at 10000 rpm for 5 min, for standby.

1.3 Analysis by liquid chromatography-mass spectrometry

### Instrument and equipment

### HPLC-MS-LTQ Orbitrap Discovery (Thermo, Germany)

### Chromatographic conditions

Column: C18 column (150 mm × 2.1 mm, 5 µm); Solvent A was 0.1% (v/v) formic acid/water, and solvent B was 0.1% (v/v) formic acid/methanol; gradient elution program: 0~3 min, 5% B, 3 ~ 36 min, 5% ~ 80% B, 36 ~ 40 min, 80% ~ 100% B, 40 ~ 45 min, 100% B, 45 ~ 50 min, 100% ~ 5% B, 50 ~ 60 min, 5% B; flow rate: 0.2 mL / min; injection volume: 20 µL.

### Mass spectrometry conditions

ESI ion source, positive ion mode for data acquisition, the mass scanning range was 50 ~ 1000 mass-to-charge (m/z). Ion source parameters ESI: sheath gas was 10, auxiliary air was 5, capillary temperature was 350°C, spray voltage was 4.5KV.

### 1.4 Data processing

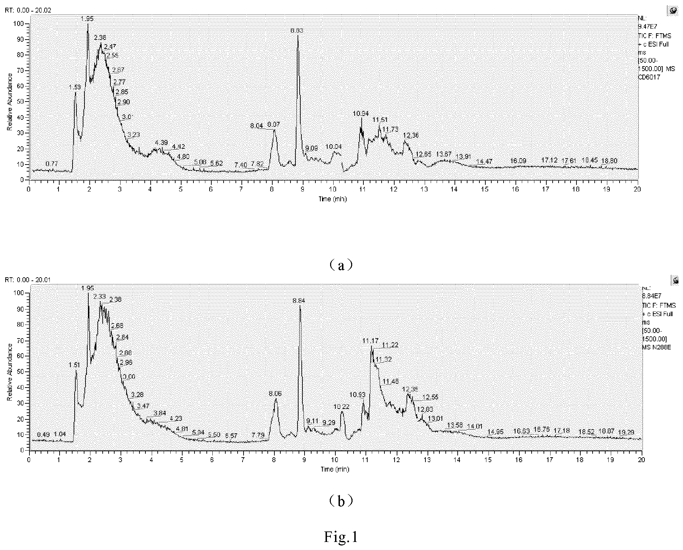

XCMS software (e.g., http://metlin.scripps.edu/xcms/) was used for peak detection and peak matching of raw data; and R software using PLS-DA (partial least squares - discriminant analysis) was used for pattern recognition analysis of differential variables of the metabolite profile of coronary heart disease group (Fig. 1a) and the metabolite profile of normal group (Fig.1b), so as to establish PLS-DA mathematical model.

### 1.5 Comparison and determination of characteristic metabolite profiles

The blood plasma metabolite profile of coronary heart disease patients (Fig. 1) was established by comparing the blood plasma metabolite profiles of the normal group and the coronary heart disease group. The results showed that there were significant differences in the blood plasma metabolite profiles between the normal group and the coronary heart disease group.

### Example 2

2.1 Sample collection: morning blood plasma samples of volunteers were collected, immediately placed and stored in -80°C low temperature refrigerator. A total of 52 blood plasma samples were collected from the normal group and 40 blood plasma samples were collected from the coronary heart disease group.

2.2 Sample treatment: frozen samples were thawed at room temperature, 500 µL of each blood plasma sample was taken and placed in 2.0 mL centrifuge tube, added with 1000 µL of methanol for dilution, centrifuged at 10000 rpm for 5 min, for standby.

2.3 Analysis by liquid chromatography-mass spectrometry

### Instrument and equipment

### HPLC-MS-LTQ Orbitrap Discovery (Thermo, Germany)

### Chromatographic conditions

Column: C18 column (150 mm × 2.1 mm, 5 µm); mobile phase A: 0.1% formic acid aqueous solution, mobile phase B: 0.1% formic acid in acetonitrile solution; gradient elution program: 0~3 min, 5% B, 3 ~ 36 min, 5% ~ 80% B, 36 ~ 40 min, 80% ~ 100% B, 40 ~ 45 min, 100% B, 45 ~ 50 min, 100% ~ 5% B, 50 ~ 60 min, 5% B; flow rate: 0.2 mL / min; injection volume: 20 µL.

### Mass spectrometry conditions

ESI ion source, positive ion mode for data acquisition, scanning mass m/z 50 ~ 1000. Ion source parameters ESI: sheath gas was 10, auxiliary air was 5, capillary temperature was 350°C, cone hole voltage was 4.5KV.

### 2.4 Data processing

XCMS software was used for relevant pretreatment of raw data to obtain a two-dimensional matrix data, and wilcox-test was used to statistically determine significant differences of peaks of metabolites; and PLS-DA (partial least squares - discriminant analysis) was used for pattern recognition analysis of differential variables of the metabolite profile of coronary heart disease group (Fig.1a) and the metabolite profile of normal group (Fig.1b), and potential biomarkers were screened out by VIP, Volcano-plot and S-plot in combination.

### 2.5 Metabolic profile analysis and potential biomarkers

### 2.5.1 Orthogonal partial least squares discriminant analysis (PLS-DA)

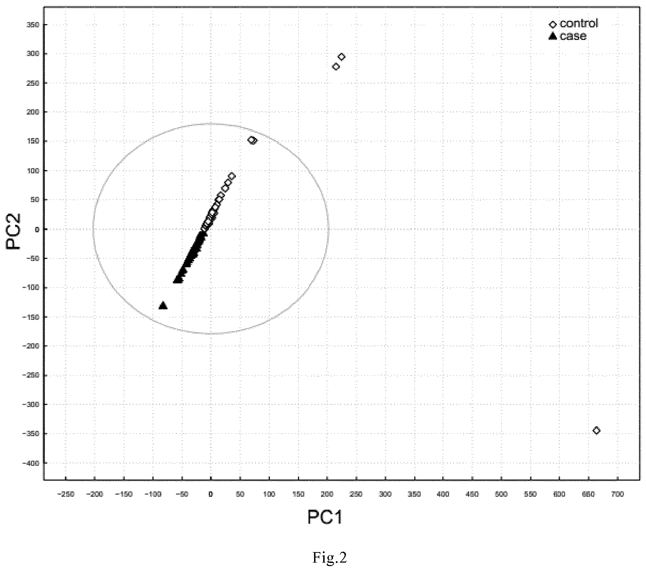

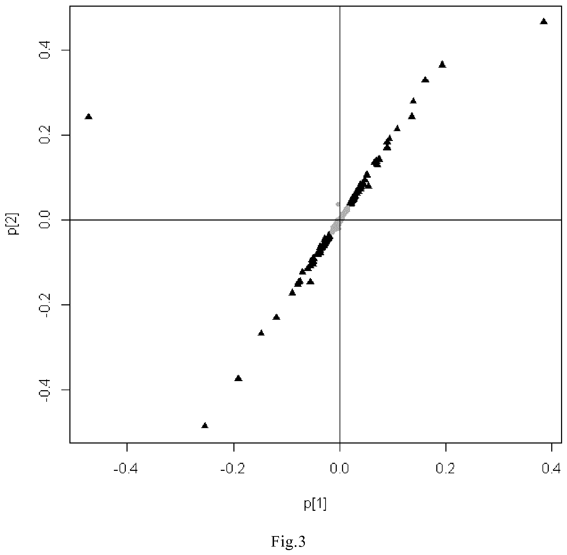

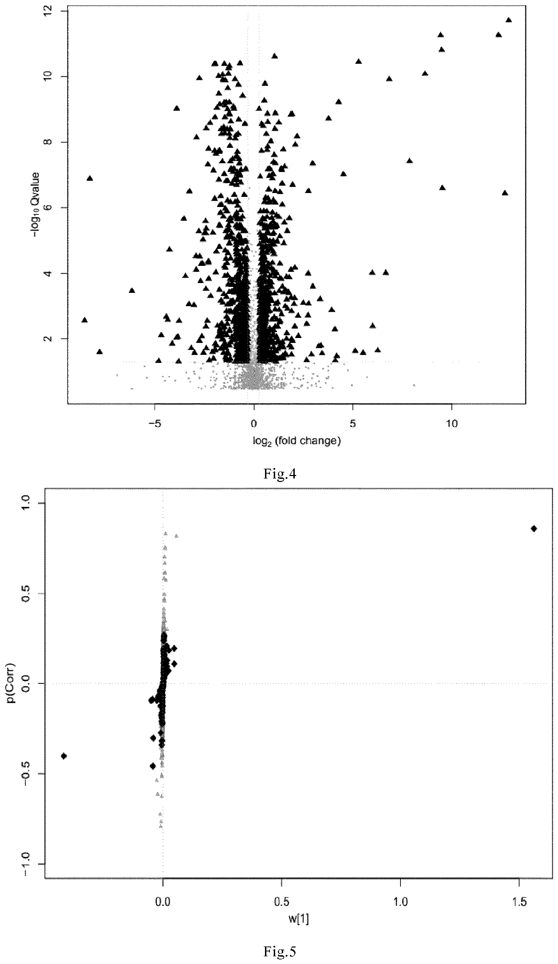

PLS-DA method was used to distinguish the normal group and the coronary heart disease group, and potential markers were further screened by VIP values (Loading-plot for principal component analysis) (Fig. 3), Volcano-plot (Fig. 4) and S-plot (Fig. 5). It was shown in Fig.3 and Fig.4 that there were significant different metabolites in the normal group and coronary heart disease group. As shown in Fig.5, each point in the S-plot represented a variable, and the S-plot graph showed the relevance of the variable to the model. The black prism-tagged variable was a variable with VIP greater than 1, which had a large deviation and a good correlation with the model (see Fig.2 and Fig.5).

### 2.5.2 Potential biomarkers

The potential markers were screened according to the VIP values of the PLS-DA model for pattern cognition. The variables with VIP values greater than 1 were extracted from the PLS-DA model, and variables with large deviation and relevance were further selected according to Loading-plot, Volcano-plot and S-plot, and 6 potential biomarkers were obtained by further combining variables with P value of less than 0.05 and Q value of less than 0.05, which were shown in Table 1.

**Table 1: Potential biomarkers**

| Mass- to-charge ratio (amu) | Retention time, Rt (sec) | Ratio (normal group/coronary heart disease group) | P value | Q value | VIP value |
|---|---|---|---|---|---|
| 310.04 | 611.25 | 0.0001 | 2.17E-15 | 1.92E-12 | 1.16 |
| 311.05 | 611.26 | 0.0025 | 1.35E-12 | 8.20E-11 | 1.16 |
| 220.00 | 122.77 | 0.6414 | 3.86E-02 | 2.75E-02 | 1.01 |
| 247.09 | 146.37 | 0.3984 | 5.61E-08 | 3.37E-07 | 1.24 |
| 255.03 | 117.92 | 0.3484 | 3.83E-06 | 1.21E-05 | 1.26 |
| 170.03 | 202.18 | 0.0156 | 3.79E-03 | 4.08E-03 | 4.54 |

### 2.5.3 Principal Component Analysis (PCA)

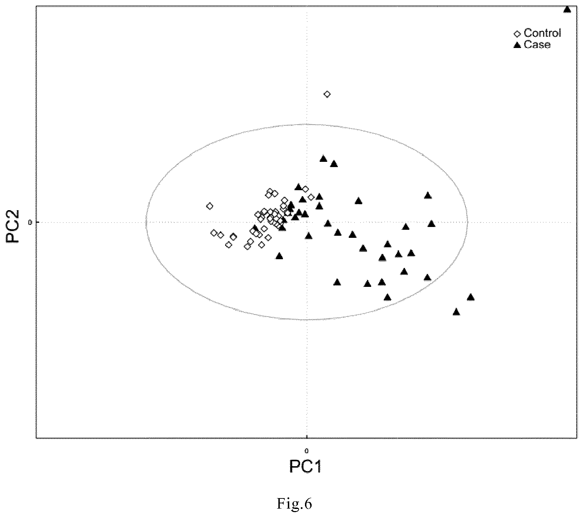

PCA is a non-supervised pattern recognition method that can visually describe differences between samples in multidimensional space. PCA analysis was performed on 83 samples of the obese group and control group using the resultant six differential markers. It can be seen from Fig.6, in the PCA model, the two groups were substantially divided in the first principal component orientation, indicating there were significant differences in blood plasma metabolic profiles between the normal group and the coronary heart disease group.

### 2.5.4 Receiver operating characteristic curve (ROC)

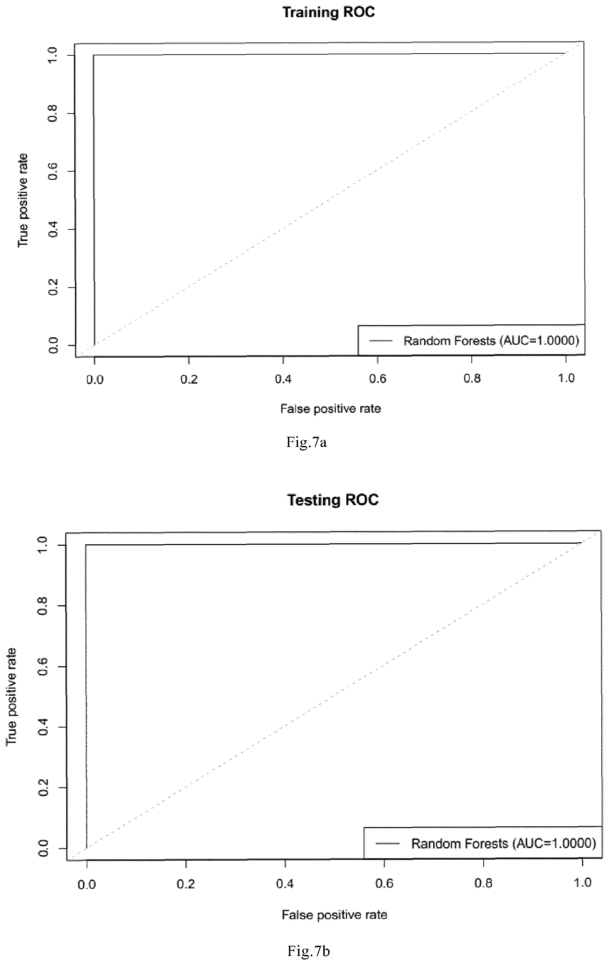

The six potential markers were discriminated in the normal group and the coronary heart disease group by using a random forest model (Random Forest)^{[7]} and receiver operating characteristic curve (ROC)^{[8]}. The data of peak areas of 92 metabolite profiles of the normal group and the coronary heart disease group were selected and used as training set via ROC modeling (see references [7] and [8]) (Fig.7a, Table 2). In addition, 83 test samples (including 38 coronary heart disease samples and 45 normal control samples) were selected as test set(Fig.7b). The test results showed AUC = 1, FN (false negative) = 0, FP (false positive) = 0 (Fig.7). Thus, the present invention has high accuracy and specificity, and has good prospects to be developed as a diagnosis method to provide a basis for diagnosis of coronary heart disease.

**Table 2: Peak area data of training set metabolite profiles**

| Sample No. | Group (1: Coronary heart disease group; 0: normal group) | Mass-to-charge ratio | | | | | |
|---|---|---|---|---|---|---|---|
| | | 310.0474 | 311.0511 | 220.0088 | 247.0927 | 255.0378 | 170.0328 |
| CD5751_1 | 1 | 0.035971 | 0.028941 | 2.0698 | 1.847557 | 3.447373 | 1.838497 |
| CD5767_1 | 1 | 0.518048 | 0.528351 | 1.67918 | 1.057417 | 1.427185 | 0.642363 |
| CD5778_1 | 1 | 0.118419 | 0.114578 | 5.274362 | 1.636753 | 5.136574 | 0.856236 |
| CD5779_1 | 1 | 0.830761 | 0.825451 | 1.981177 | 1.820087 | 0.678725 | 3.258685 |
| CD5782_1 | 1 | 2.273 | 2.266954 | 2.183817 | 1.795206 | 2.589921 | 0.378109 |
| CD5783_1 | 1 | 0.142664 | 0.143573 | 1.950855 | 1.546727 | 1.105065 | 1.232417 |
| CD5788_1 | 1 | 0.297865 | 0.296303 | 0.80925 | 0.357977 | 0 | 0.163506 |
| CD5795_1 | 1 | 5.468021 | 5.71178 | 1.796937 | 1.627936 | 2.73744 | 0.247029 |
| CD5796_1 | 1 | 0.218944 | 0.217782 | 3.1631 | 1.449651 | 2.229824 | 0.525126 |
| CD5797_1 | 1 | 1.4614 | 1.436343 | 3.226244 | 0.713308 | 2.545898 | 0.692436 |
| CD5805_1 | 1 | 0.79711 | 0.813508 | 2.906224 | 1.529678 | 0.3218 | 1.169494 |
| CD5814_1 | 1 | 2.094091 | 2.036124 | 1.027309 | 0.67968 | 0.706373 | 0.526758 |
| CD5816_1 | 1 | 2.73073 | 2.79561 | 1.510684 | 1.580599 | 1.310362 | 0.566224 |
| CD5819_1 | 1 | 0.001538 | 0.01335 | 1.591497 | 1.396263 | 0.941163 | 0 |
| CD5822_1 | 1 | 3.744198 | 3.809473 | 2.267795 | 1.429436 | 2.422802 | 0.086586 |
| CD5831_1 | 1 | 3.260472 | 3.348123 | 2.334711 | 1.218704 | 3.769648 | 1.490568 |
| CD5832_1 | 1 | 0.028251 | 0.024733 | 1.883654 | 1.928723 | 4.108324 | 0.871313 |
| CD5833_1 | 1 | 3.207548 | 3.22654 | 1.264351 | 0.833532 | 5.375295 | 0.26837 |
| CD5838_1 | 1 | 0.672543 | 0.669593 | 2.269154 | 1.465057 | 1.48128 | 1.196006 |
| CD5851_1 | 1 | 2.225022 | 2.232207 | 2.231923 | 1.564476 | 1.626777 | 1.065598 |
| CD5860_1 | 1 | 3.588629 | 3.619216 | 1.623603 | 0.349931 | 1.466329 | 2.37164 |
| CD5863_1 | 1 | 0.01132 | 0.011593 | 2.806604 | 1.334728 | 1.423662 | 0.469683 |
| CD5867_1 | 1 | 0.096234 | 0.095499 | 1.864931 | 1.113036 | 4.784302 | 0.359933 |
| CD5871_1 | 1 | 3.275621 | 3.38236 | 1.351764 | 0.405 | 2.682353 | 0.089361 |
| CD5877_2 | 1 | 2.900862 | 2.903243 | 6.918055 | 0.863341 | 1.525894 | 0.248852 |
| CD5881_2 | 1 | 4.546949 | 4.59075 | 6.97697 | 0.302554 | 1.321564 | 1.111718 |
| CD5884_2 | 1 | 0.457603 | 0.461464 | 9.928549 | 2.066072 | 12.84567 | 0.449829 |
| CD5891_2 | 1 | 0.098234 | 0.106791 | 0.84521 | 1.595202 | 2.156983 | 0.800175 |
| CD5892_2 | 1 | 0 | 0.000933 | 5.960872 | 1.560398 | 3.367482 | 0.560264 |
| CD5898_2 | 1 | 0.000153 | 0.004361 | 0.817571 | 1.463604 | 1.986249 | 0.713979 |
| CD5900_2 | 1 | 2.296194 | 2.291767 | 6.686202 | 0.650392 | 1.617971 | 0.69235 |
| CD5916_2 | 1 | 0.229847 | 0.112529 | 7.224221 | 1.473773 | 1.725251 | 0.841159 |
| CD5923_2 | 1 | 1.470656 | 1.481594 | 11.02546 | 1.473311 | 1.955908 | 1.359043 |
| CD5925_2 | 1 | 0.000292 | 0 | 5.76178 | 1.351079 | 1.62615 | 0.127491 |
| CD5926_2 | 1 | 0.000984 | 0.002923 | 0.668754 | 1.208468 | 1.655 | 0.554825 |
| CD5931_2 | 1 | 3.045312 | 3.090104 | 0.555586 | 0.246728 | 3.868446 | 0.107213 |
| CD5934_2 | 1 | 2.761015 | 2.807205 | 0.932432 | 1.439827 | 2.482806 | 0.332513 |
| CD5935_2 | 1 | 2.59472 | 2.570944 | 6.481325 | 1.600715 | 1.829884 | 0.653901 |
| CD5988_2 | 1 | 0.074743 | 0.076354 | 8.927902 | 0.287215 | 4.092279 | 0.856305 |
| CD5990_2 | 1 | 0.528431 | 0.531329 | 7.455305 | 2.119379 | 8.704949 | 0.402132 |
| N165E_2 | 0 | 0 | 0.001198 | 2.273735 | 0.219128 | 1.404477 | 0.468192 |
| N167E_2 | 0 | 0.000153 | 0.003134 | 0.330128 | 1.427848 | 2.519408 | 0.947302 |
| N168E_2 | 0 | 0 | 0.004276 | 0.532362 | 1.193247 | 2.307861 | 0.635855 |
| N170E_2 | 0 | 0.000123 | 0.006678 | 1.865563 | 0.62836 | 1.752023 | 0.876193 |
| N171E_2 | 0 | 0.000108 | 0.002391 | 0.69803 | 1.43365 | 1.397692 | 0.506712 |
| N185E_2 | 0 | 0 | 0.000993 | 0.556966 | 1.31429 | 1.097642 | 0.570415 |
| N186E_2 | 0 | 0 | 0.000913 | 0.315125 | 1.586638 | 0.800838 | 0.930858 |
| N187E_2 | 0 | 0 | 0.004406 | 2.299563 | 0.286376 | 1.737154 | 1.048589 |
| N190E_2 | 0 | 0.0043 | 0.002773 | 1.338285 | 1.36053 | 1.854572 | 1.10703 |
| N191E_2 | 0 | 0 | 0.002541 | 4.508579 | 1.504058 | 4.033288 | 0.69268 |
| N195E_2 | 0 | 0 | 0.004779 | 0.385074 | 0.251441 | 0.94138 | 1.314871 |
| N197E_2 | 0 | 0 | 0.002084 | 2.091143 | 1.331258 | 1.345158 | 0.77953 |
| N198gan_huruilian_2 | 0 | 0.000116 | 0.003454 | 4.933436 | 0.018222 | 0 | 1.500137 |
| N199gan_linrufang_2 | 0 | 0 | 0.001958 | 2.272987 | 0.022897 | 0 | 1.087806 |
| N200E_2 | 0 | 0 | 0.000896 | 0.427487 | 0.433903 | 2.398669 | 0.340811 |
| N201gan_lvhuiX_2 | 0 | 0 | 0.002775 | 2.366315 | 0.013556 | 0.035438 | 0.362495 |
| N203gan_1 | 0 | 0 | 0.003349 | 0.887285 | 0.078262 | 0 | 3.139258 |
| N204gan_wangmiaorong_1 | 0 | 0.000167 | 0.00143 | 0.909609 | 0.06722 | 0.104947 | 2.562048 |
| N205gan_liuqifang_1 | 0 | 0 | 0 | 0.853212 | 0.049627 | 0.049099 | 3.671745 |
| N206gan_liuguoying_1 | 0 | 0.000268 | 0.002434 | 0.860768 | 0.071575 | 0 | 3.307561 |
| N207E_2 | 0 | 0.001081 | 0.002212 | 0.662516 | 0.635003 | 2.114432 | 0.689146 |
| N208gan_zhengshuX_2 | 0 | 0 | 0.00256 | 4.877083 | 0.023979 | 0 | 0.575836 |
| N209gan_wangxin_2 | 0 | 9.43E-05 | 0.00151 | 1.282423 | 0.013061 | 0 | 1.529018 |
| N212E_1 | 0 | 0.000208 | 0.006068 | 1.451385 | 1.210609 | 0.883391 | 2.824106 |
| N213E_1 | 0 | 0 | 0.003607 | 0.213409 | 1.329888 | 0.812546 | 0.567079 |
| N214E_1 | 0 | 0.000194 | 0.002669 | 0.345 | 1.358322 | 0.37904 | 2.964935 |
| N215E_1 | 0 | 0 | 0.001307 | 0.616592 | 2.579593 | 1.53582 | 0.878895 |
| N217E_1 | 0 | 0.000261 | 0.002067 | 1.393546 | 1.469455 | 0.781215 | 2.802722 |
| N218E_1 | 0 | 0.000154 | 0 | 1.002086 | 0.994796 | 1.424977 | 0.515163 |
| N220E_1 | 0 | 0 | 0.002992 | 0.836052 | 1.463508 | 0.756875 | 3.049891 |
| N222E_1 | 0 | 0 | 0.00519 | 1.476197 | 1.434959 | 0.853225 | 1.892389 |
| N223E_1 | 0 | 0 | 0.002726 | 0.994038 | 1.259697 | 0.732209 | 2.262991 |
| N226E_1 | 0 | 0 | 0.033521 | 0.245588 | 1.383716 | 1.351605 | 0.71867 |
| N227E_1 | 0 | 0 | 0.006596 | 1.112362 | 1.38151 | 1.48167 | 0.675359 |
| N228E_1 | 0 | 0.001776 | 0.001104 | 2.128 | 0.720528 | 0.740941 | 1.334337 |
| N229E_1 | 0 | 0 | 0.006393 | 1.382065 | 0.957774 | 0.537195 | 2.750898 |
| N231E_1 | 0 | 0 | 0.002535 | 0.864953 | 1.816202 | 2.984069 | 2.654364 |
| N232E_1 | 0 | 0 | 0.002986 | 0.396396 | 0.411945 | 0.42109 | 4.548935 |
| N233E_1 | 0 | 0 | 0.006421 | 1.02865 | 0.464872 | 2.490481 | 1.610518 |
| N234E_1 | 0 | 0 | 0.004642 | 1.071868 | 1.526404 | 1.642752 | 0.63371 |
| N235E_1 | 0 | 0 | 0 | 1.634843 | 0.480648 | 1.674944 | 0.985628 |
| N236E_1 | 0 | 0 | 0.004328 | 1.166732 | 0.867571 | 1.2451 | 1.58132 |
| N237E_1 | 0 | 0.000284 | 0.002743 | 1.382313 | 0.273098 | 0.619799 | 5.461519 |
| N238E_1 | 0 | 0 | 0.002365 | 0.138054 | 1.218771 | 0.687922 | 1.281134 |
| N239E_1 | 0 | 0.000128 | 0.005445 | 3.55656 | 1.314373 | 0.736699 | 0.753425 |
| N241E_1 | 0 | 0.000128 | 0.00495 | 1.320156 | 0.331016 | 0.915875 | 1.332363 |
| N242E_1 | 0 | 0.000169 | 0.003374 | 1.959169 | 0.166331 | 0.319434 | 1.013391 |
| N243E_1 | 0 | 0 | 0.001174 | 1.286338 | 1.603114 | 1.067129 | 2.545529 |
| N244E_1 | 0 | 0 | 0.020157 | 0.885433 | 1.110168 | 1.142692 | 1.858838 |
| N245E_1 | 0 | 0.000156 | 0.001082 | 0.237614 | 0.360275 | 0.671792 | 2.873411 |
| N247E_2 | 0 | 0.000107 | 0.004216 | 0.247196 | 1.321152 | 0.972867 | 0.926548 |
| N248E_2 | 0 | 0 | 0.001756 | 0.264963 | 1.250571 | 0.716003 | 1.141921 |

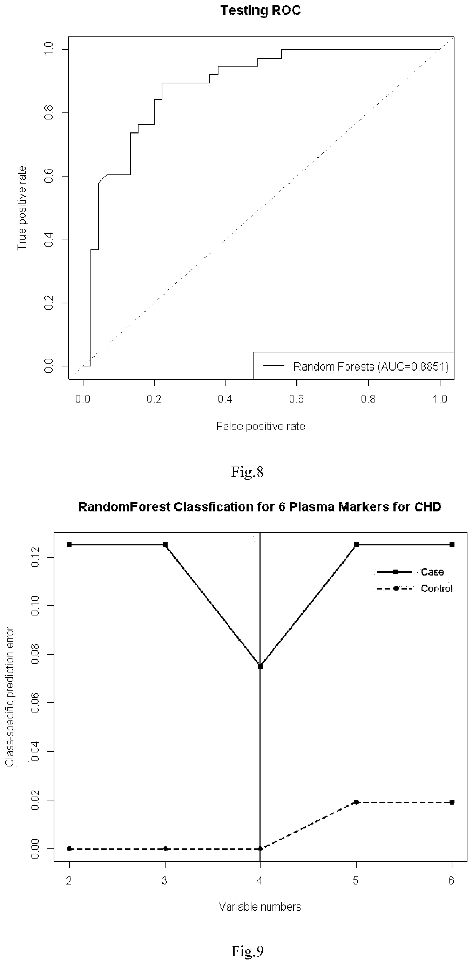

Using the random forest model to calculate the classification ability of the six potential biomarkers for the coronary heart disease group and the normal group, the results of the classification ability (arranged from high to low) were shown in Table 3. The markers in the table should be tested using at least above 4 markers (Fig.9), so as to maintain high sensitivity and specificity.

**Table 3: Classification ability of potential biomarkers**

| Metabolite (mass- to-charge ratio) (amu) | Interpreting value of normal group | Interpreting value of coronary heart disease group | Mean Decrease Accuracy | Mean Decrease Gini |
|---|---|---|---|---|
| 310.04 | 0.168808 | 0.203038 | 0.180087 | 17.56405 |
| 311.05 | 0.111137 | 0.137121 | 0.119923 | 14.38203 |
| 220.00 | 0.023173 | 0.020618 | 0.021529 | 3.634677 |
| 170.03 | 0.017363 | 0.015979 | 0.016477 | 3.425181 |
| 255.03 | 0.010633 | 0.005938 | 0.008497 | 3.596366 |
| 247.09 | 0.007744 | 0.006723 | 0.007192 | 2.095741 |

If mass-to-charge ratios, such as 310.04 and 311.05, were randomly removed from the training set, the resultant ROC test set (the above 83 test set samples) had AUC = 0.8851, AUC decreased significantly, FN = 0.184 and FP = 0.200, FN and FP significantly increased (Fig.8), which indicated the ability for diagnosis of coronary heart disease decreased.

### References:

[1] Finegold, JA; Asaria, P; Francis, DP. Mortality from ischaemic heart disease by country, region, and age: Statistics from World Health Organisation and United Nations. International journal of cardiology. 4 December 2012, 168 (2): 934-45.
[2] World Health Organization Department of Health Statistics and Informatics in the Information, Evidence and Research Cluster. The global burden of disease 2004 update. Geneva: WHO. 2004. ISBN 92-4-156371-0.
[3] Elizabeth Barrett-Connor. Gender differences and disparities in all-cause and coronary heart disease mortality: epidemiological aspects. Best Pract Res Clin Endocrinol Metab. 2013 Aug;27(4):481-500.
[4] Madjid M, Willerson JT. Inflammatory markers in coronary heart disease. Br Med Bull. 2011;100:23-38. doi: 10.1093/bmb/ldr043. Epub 2011 Oct 18.
[5] Spoletini Il, Vitale C, Rosano GM. Biomarkers for predicting postmenopausal coronary heart disease. Biomark Med. 2011 Aug;5(4):485-95. doi: 10.2217/bmm.11.51.
[6] Kishore Kumar Pasikanti, Kesavan Esuvaranathan, Paul C. Ho, et al. Noninvasive urinary metabonomic diagnosis of human bladder cancer. Journal of Proteome Research, 2010, 9, 2988-2995.
[7] Liaw, Andy & Wiener, Matthew. Classification and Regression by randomForest, R News (2002), Vol. 2/3 p. 18.
[8] Jianguo Xia, David I. Broadhurst, Michael Wilson, David S. Wishart. Translational biomarker discovery in clinical metabolomics: an introductory tutorial. Metabolomics (2013) 9:280-299.

## Claims

1. A method for risk assessment, diagnosis, early diagnosis or pathological staging of coronary heart disease, comprising the following steps:
(1) determining content of each biomarker of a biomarker composition in a blood plasma sample or a whole blood sample of a subject;
wherein the biomarker composition comprises at least one or more selected from the following Biomarkers 1 to 6:
Biomarker 1, which has a mass-to-charge ratio of 310.04 ± 0.4amu, and a retention time of 611.25 ± 60s;
Biomarker 2, which has a mass-to-charge ratio of 311.05 ± 0.4amu, and a retention time of 611.26 ± 60s;
Biomarker 3, which has a mass-to-charge ratio of 220.00 ± 0.4amu, and a retention time of 122.77 ± 60s;
Biomarker 4, which has a mass-to-charge ratio of 247.09 ± 0.4amu, and a retention time of 146.37 ± 60s;
Biomarker 5, which has a mass-to-charge ratio of 255.03 ± 0.4amu, and a retention time of 117.92 ± 60s; and
Biomarker 6, which has a mass-to-charge ratio of 170.03 ± 0.4amu, and a retention time of 202.18 ± 60s;
Wherein the content of each biomarker is determined via a liquid chromatography-mass spectrometry method;
(2) establishing a training set for content of each biomarker of the biomarker composition according to step (1) of a coronary heart disease subject and a training set for content of each biomarker of the biomarker composition according to step (1) of a normal subject;
(3) comparing the content of each biomarker of a subject in step (1) to data of the training set in step (2) using a receiver operating characteristic curve; and
(4) interpring the result of step (3).
wherein the content of each biomarker of a biomarker composition in a blood plasma sample or a whole blood sample of a subject is determined by liquid chromatography-mass spectrometry;
wherein chromatographic conditions are as follows:
Column: C18 column; Solvent A was 0.1% (v/v) formic acid/water, and solvent B was 0.1% (v/v) formic acid/methanol; gradient elution program: 0-3 min, 5% B, 3-36 min, 5%- 80% B, 36 - 40 min, 80% - 100% B, 40 - 45 min, 100% B, 45 - 50 min, 100% - 5% B, 50 - 60 min, 5% B; flow rate: 0.2 mL / min; injection volume: 20 µL;
wherein mass spectrometry conditions are as follows:
ESI ion source, positive ion mode for data acquisition, the mass scanning range was 50 - 1000 mass-to-charge, Ion source parameters ESI: sheath gas was 10, auxiliary air was 5, capillary temperature was 350°C, spray voltage was 4.5KV.

2. The method according to claim 1, wherein the biomarker composition comprises at least Biomarkers 1 to 3 and 6.

3. The method according to claim 2, wherein the biomarker composition further comprises Biomarker 4 and/or Biomarker 5.

4. The method according to claim 1, wherein the biomarker composition comprises Biomarkers 1 to 6.

5. The method according to any one of claims 1-4, wherein the training set in step (2) is established by using a multivariate statistical classification model.

6. The method according to claim 5, wherein the multivariate statistical classification model is a random forest model.

## Patentansprüche

1. Verfahren für Risikobewertung, Diagnose, Früherkennung oder pathologisches Staging von koronarer Herzerkrankung, das die folgenden Schritte umfasst:
(1) Bestimmen des Gehalts an jedem Biomarker einer Biomarker-Zusammensetzung in einer Blutplasmaprobe oder einer Vollblutprobe von einem Individuum;
wobei die Biomarker-Zusammensetzung mindestens einen oder mehrere, ausgewählt aus den folgenden Biomarkern 1 bis 6, umfasst:
Biomarker 1, der ein Masse-zu-Ladung-Verhältnis von 310,04 ± 0,4 amu und eine Retentionszeit von 611,25 + 60 s aufweist;
Biomarker 2, der ein Masse-zu-Ladung-Verhältnis von 311,05 ± 0,4 amu und eine Retentionszeit von 611,26 + 60 s aufweist;
Biomarker 3, der ein Masse-zu-Ladung-Verhältnis von 220,00 ± 0,4 amu und eine Retentionszeit von 122,77 + 60 s aufweist;
Biomarker 4, der ein Masse-zu-Ladung-Verhältnis von 247,09 ± 0,4 amu und eine Retentionszeit von 146,37 + 60 s aufweist;
Biomarker 5, der ein Masse-zu-Ladung-Verhältnis von 255,03 ± 0,4 amu und eine Retentionszeit von 117,92 + 60 s aufweist; und
Biomarker 6, der ein Masse-zu-Ladung-Verhältnis von 170,03 ± 0,4 amu und eine Retentionszeit von 202,18 + 60 s aufweist;
wobei der Gehalt an jedem Biomarker mittels eines Flüssigchromatographie-Massenspektrometrie-Verfahrens bestimmt wird;
(2) Festlegen eines Trainingssatzes für den Gehalt an jedem Biomarker der Biomarker-Zusammensetzung gemäß Schritt (1) von einem Individuum mit koronarer Herzerkrankung und eines Trainingssatzes für den Gehalt an jedem Biomarker der Biomarker-Zusammensetzung gemäß Schritt (1) von einem gesunden Individuum;
(3) Vergleichen des Gehalts an jedem Biomarker von einem Individuum in Schritt (1) mit Daten des Trainingssatzes in Schritt (2) unter Verwendung einer Receiver Operating Characteristic-Kurve; und
(4) Interpretieren des Ergebnisses aus Schritt (3);
wobei der Gehalt an jedem Biomarker einer Biomarker-Zusammensetzung in einer Blutplasmaprobe oder einer Vollblutprobe von einem Individuum mittels Flüssigchromatographie-Massenspektrometrie bestimmt wird;
wobei die chromatographischen Bedingungen wie folgt sind:
Säule: C18-Säule; Lösungsmittel A war 0,1 % (v/v) Ameisensäure/Wasser, und Lösungsmittel B war 0,1 % (v/v) Ameisensäure/Methanol;
Gradientenelutionsprogramm: 0-3 min, 5 % B, 3-36 min, 5 %-80 % B, 36-40 min, 80 %-100 % B, 40-45 min, 100 % B, 45-50 min, 100 %-5 % B, 50-60 min, 5 % B; Flussrate: 0,2 ml/min; Injektionsvolumen: 20 µl;
wobei die massenspektrometrischen Bedingungen wie folgt sind:
ESI-Ionenquelle, positiver Ionenmodus für Datenerfassung, der Massen-Scanning-Bereich war 50-1000 Masse-zu-Ladung, Ionenquellen-Parameter ESI: Hüllgas war 10, Zusatzluft war 5, Kapillartemperatur war 350 °C, Sprühspannung war 4,5 KV.

2. Verfahren gemäß Anspruch 1, wobei die Biomarker-Zusammensetzung zumindest die Biomarker 1 bis 3 und 6 umfasst.

3. Verfahren gemäß Anspruch 2, wobei die Biomarker-Zusammensetzung weiterhin Biomarker 4 und/oder Biomarker 5 umfasst.

4. Verfahren gemäß Anspruch 1, wobei die Biomarker-Zusammensetzung die Biomarker 1 bis 6 umfasst.

5. Verfahren gemäß einem der Ansprüche 1-4, wobei der Trainingssatz in Schritt (2) unter Verwendung eines multivariaten statistischen Klassifikationsmodells festgelegt wird.

6. Verfahren gemäß Anspruch 5, wobei das multivariate statistische Klassifikationsmodell ein Random-Forest-Modell ist.

## Revendications

1. Procédé d'évaluation de risque, de diagnostic, de diagnostic précoce ou de stadification pathologique d'une maladie coronarienne, comprenant les étapes suivantes :
(1) déterminer la teneur en chaque biomarqueur d'une composition de biomarqueurs dans un échantillon de plasma sanguin ou un échantillon de sang total d'un sujet ;
dans lequel la composition de biomarqueurs comprend au moins un ou plusieurs sélectionnés parmi les biomarqueurs 1 à 6 suivants :
biomarqueur 1, qui a un rapport masse sur charge de 310,04 ± 0,4 amu, et un temps de rétention de 611,25 ± 60 s ;
biomarqueur 2, qui a un rapport masse sur charge de 311,05 ± 0,4 amu, et un temps de rétention de 611,26 ± 60 s ;
biomarqueur 3, qui a un rapport masse sur charge de 220,00 ± 0,4 amu, et un temps de rétention de 122,77 ± 60 s ;
biomarqueur 4, qui a un rapport masse sur charge de 247,09 ± 0,4 amu, et un temps de rétention de 146,37 ± 60 s ;
biomarqueur 5, qui a un rapport masse sur charge de 255,03 ± 0,4 amu, et un temps de rétention de 117,92 ± 60 s ;
biomarqueur 6, qui a un rapport masse sur charge de 170,03 ± 0,4 amu, et un temps de rétention de 202,18 ± 60 s ;
dans lequel la teneur en chaque biomarqueur est déterminée par l'intermédiaire d'un procédé de chromatographie en phase liquide - spectrométrie de masse ;
(2) l'établissement d'un jeu d'entraînement pour la teneur en chaque biomarqueur de la composition de biomarqueurs conformément à l'étape (1) d'un sujet souffrant d'une maladie coronarienne et d'un jeu d'entraînement pour la teneur en chaque biomarqueur de la composition de biomarqueurs conformément à l'étape (1) d'un sujet normal ;
(3) la comparaison de la teneur en chaque biomarqueur d'un sujet à l'étape (1) aux données du jeu d'entraînement à l'étape (2) en utilisant une courbe ROC (receiver operating characteristic curve) ; et
(4) l'interprétation du résultat de l'étape (3) ;
dans lequel la teneur en chaque biomarqueur d'une composition de biomarqueurs dans un échantillon de plasma sanguin ou un échantillon de sang total d'un sujet est déterminée par chromatographie en phase liquide - spectrométrie de masse ;
dans lequel les conditions chromatographiques sont les suivantes :
colonne : colonne C18 ; le solvant A était acide formique à 0,1 % (v/v)/eau, et le solvant B était acide formique à 0,1 % (v/v)/méthanol ; le programme d'élution en gradient était : 0 à 3 min, 5 % de B, 3 à 36 min, 5 % à 80 % de B, 36 à 40 min, 80 % à 100 % de B, 40 à 45 min, 100 % de B, 45 à 50 min, 100 % à 5 % de B, 50 à 60 min, 5 % de B ; débit : 0,2 ml/min ; volume d'injection : 20 µl ;
dans lequel les conditions de spectrométrie de masse sont les suivantes :
source d'ions ESI, mode ion positif pour l'acquisition de données, la plage de balayage de masse était de 50 à 1000 masse/charge, paramètres de source d'ions ESI : le gaz de gaine était 10, l'air auxiliaire était 5, la température du capillaire était de 350 °C, la tension de nébulisation était de 4,5 KV.

2. Procédé selon la revendication 1, dans lequel la composition de biomarqueurs comprend au moins les biomarqueurs 1 à 3 et 6.

3. Procédé selon la revendication 2, dans lequel la composition de biomarqueurs comprend en outre le biomarqueur 4 et/ou le biomarqueur 5.

4. Procédé selon la revendication 1, dans lequel la composition de biomarqueurs comprend les biomarqueurs 1 à 6.

5. Procédé selon l'une quelconque des revendications 1 à 4, dans lequel le jeu d'entraînement à l'étape (2) est établi en utilisant un modèle de classification statistique à plusieurs variables.

6. Procédé selon la revendication 5, dans lequel le modèle de classification statistique à plusieurs variables est un modèle de forêt aléatoire.
